# EUROPÄISCHE PATENTSCHRIFT

(11) **EP 1 628 570 B1**
(45) Veröffentlichungstag und Bekanntmachung des Hinweises auf die Patenterteilung: **11.04.2007**
(21) Anmeldenummer: 05715010.4
(22) Anmeldetag: 16.02.2005
(51) Int. Cl.: A61B 5/0215

(54) **VERFAHREN ZUR BESTIMMUNG HÄMODYNAMISCHER PARAMETER**
METHOD FOR THE DETERMINATION OF HEMODYNAMIC PARAMETERS
PROCEDE POUR DETERMINER DES PARAMETRES HEMODYNAMIQUES

(30) Priorität: 26.02.2004 DE 102004009871
(43) Veröffentlichungstag der Anmeldung: 01.03.2006
(73) Patentinhaber: Deutsches Herzzentrum Berlin, 13353 Berlin (DE)
(72) Erfinder: WELLNHOFER, Ernst, 10551 Berlin (DE)
(74) Vertreter: Ninnemann, Detlef
(86) Internationale Anmeldenummer: PCT/DE2005/000304
(87) Internationale Veröffentlichungsnummer: WO 2005/082243

(56) Entgegenhaltungen:
- DE-A- 10 049 734
- DE-A- 19 820 844
- FR-A- 2 868 936

## Beschreibung

Die Erfindung bezieht sich auf ein Verfahren und eine Vorrichtung zur Bestimmung hämodynamischer Parameter aus Patientendrucksignalen.

Zur invasiven intraarteriellen und intravenösen Druckmessung werden in der Kardiologie, Intensivmedizin und Anästhesie Übertragungssysteme eingesetzt, bei denen die Druckmessung im Körper eines Patienten erfolgt und über das beispielsweise als Katheter ausgebildete Übertragungssystem zu einem außerhalb des Patientenkörpers angeordneten Speichermittel übertragen wird.

Es besteht dabei die Notwendigkeit, die übertragenen Daten sowohl zu speichern als auch zu analysieren. Insbesondere ist es von Interesse, aus den eingehenden Daten hämodynamische Parameter zu bestimmen, auf deren Grundlage ein Arzt eine Diagnose erstellen kann.

Dabei tritt häufig das Problem auf, dass die eingehenden, aus dem Körper des Patienten stammenden Signale mit Fehlern behaftet sind (Artefakte). Solche Fehler entstehen insbesondere in Abhängigkeit von der Länge, dem Querschnitt, dem Aufbau und den Eigenschaften des Kathetermaterials. Es kann daher zu Resonanzen, Dämpfungen und Energieverlusten des erfassten Druckmesswertes kommen, was letztlich zu einer Verfälschung des Patientensignals führt.

Ein Verfahren zur Korrektur von solchen Artefakten, die durch das Übertragungssystem bedingt sind, ist aus der DE 198 20 844 A1 bekannt; ein Verfahren zur Ermittlung und Überwachung der Transferfunktion des Übertragungssystems ist in der DE 100 49 734 A1 beschrieben.

Es treten aber häufig auch Artefakte auf, die nicht durch Übertragungsfehler bedingt sind, wie z. B. Bewegungsartefakte, die durch Manipulation am Katheter oder Anstoßen des Katheters im Gefäß oder durch Unterbrechung der Druckmessung bei Katheterwechsel oder Injektionen entstehen, sowie solche Artefakte, die durch Spülung des Systems entstehen. Die aus solchen verfälschten Patientensignalen berechneten hämodynamischen Parameter können für den Arzt somit keine geeignete Grundlage für eine Diagnose darstellen.

Der vorliegenden Erfindung liegt daher die Aufgabenstellung zugrunde, ein Verfahren und eine Vorrichtung zur Bestimmung hämodynamischer Parameter aus Patientendrucksignalen bereitzustellen, bei dem die Bestimmung nur auf der Grundlage geeigneter Signale erfolgt.

Diese Aufgabe wird durch ein Verfahren mit den Merkmalen des Anspruchs 1 und eine Vorrichtung mit den Merkmalen des Anspruchs 10 gelöst.

Das erfindungsgemäße Verfahren zur Bestimmung hämodynamischer Parameter aus Patientendrucksignalen umfasst die folgenden Schritte:
a) Empfangen von Patientensignalen von einem Messwertempfänger, dem im Körper eines Patienten gemessene Gefäßdrücke zugeführt werden,
b) Auswahl und Bestimmung eines Patientendrucksignals als Referenzdrucksignal aus den vom Messwertempfänger übermittelten Patientendrucksignalen,
c) Unterteilung der Patientendrucksignale und des Referenzdrucksignals in Segmente,
d) Prüfung dieser Segmente auf Artefakte mit Verfahren im Zeit- und Frequenzbereich unter Verwendung der ersten Ableitung der Drucksignale nach der Zeit, wobei artefaktbehaftete Segmente verworfen werden,
e) Bestimmung von Beginn und Ende eines Herzschlags mit Hilfe des Referenzdrucksignals, und
f) Verwendung der nicht verworfenen Patientendrucksignalsegmente zur Berechnung von hämodynamischen Parametern.

Nach dem Empfangen von Patientensignalen durch einen Messwertempfänger gemäß Schritt a) und vor der Auswahl und Bestimmung eines Patientendrucksignals als Referenzdrucksignal aus den vom Messwertempfänger übermittelten Patientendrucksignalen gemäß Schritt b) kann gegebenenfalls eine Vorbehandlung der Signale zur Korrektur der durch das Übertragungssystem bedingten Artefakte mit Verfahren erfolgen, die in der DE 198 20 844 A1 bzw. der DE 100 49 734 A1 beschrieben sind. Dadurch werden die durch das Übertragungssystem bedingten Artefakte korrigiert.

Durch die Auswahl und Bestimmung eines Patientendrucksignals als Referenzdrucksignal gemäß Schritt b) wird gewährleistet, dass eine verlässliche und stabile Erkennung der Herzschläge erfolgt.

Die Unterteilung der Patientendrucksignale und des Referenzdrucksignals in Segmente gemäß Schritt c) erlaubt eine hochaufgelöste Überprüfung und Verarbeitung der Drucksignale in den folgenden Schritten.

Schritt d) dient der Erkennung von Bewegungsartefakten, Spülartefakten und/oder anderen groben mechanischen Artefakten, sowie von Signalunterbrechungen und Rauschartefakten für jedes Signal, einschließlich des Referenzsignals. Dazu wird die jeweilige Abweichung der Ableitung des Signals von beispielsweise einer geglätteten Kopie des jeweiligen Signals, bzw. der Unterschiede von Mittel, Minimum und Maximum, der Abweichung des Signals von einer Regressionslinie und weitere Analysen im Zeit- und Frequenzbereich untersucht. Durch die.Aussonderung grober Artefakte wird gewährleistet, dass nur geeignete Patientendrucksignalsegmente zur Berechnung von hämodynamischen Parametern herangezogen werden.

Es stehen grundsätzlich zwei Möglichkeiten zur Verfügung, Artefakte zu bestimmen, nämlich sowohl auf Segmentebene als auch auf Schlagebene.

Unter einem Segment wird dabei ein bestimmter, zu definierender Zeitabschnitt verstanden, in dem das jeweilige Drucksignal betrachtet wird. Dabei kann ein Segment beispielsweise 500 ms umfassen. Einer Artefaktbestimmurig auf Schlagebene liegt demgegenüber der Zeitabschnitt zugrunde, der einer Herzschlagperiode entspricht.

Die zeitliche Festlegung des Beginns und des Endes eines Herzschlags mit Hilfe des Referenzdrucksignals gemäß Schritt e) ermöglicht die lokale Suche des Beginns und des Endes des korrespondierenden Herzschlags von anderen Patientendrucksignalen.

Die nicht verworfenen Patientendrucksignalsegmente können nunmehr nach Schritt f) der Berechnung von hämodynamischen Parametern dienen, die auf unverfälschten Druckmessdaten beruhen.

Wie im Folgenden beschrieben wird, können mit Hilfe des erfindungsgemäßen Verfahrens verschiedene hämodynamische Parameter berechnet werden: Insbesondere erfolgt auf Grundlage der Patientendrucksignale und/oder der Referenzdrucksignale eine Signalartbestimmung, eine Herzzeitvolumenbestimmung, eine Schlagbestimmung am Referenzsignal, eine signalarttypische Auswertung und eine Niederfrequenzanalyse (der sogenannten Mayerwellen).

### 1. Schlagbestimmung am Referenzsignal

Eine signalartabhängige Schwellwertbestimmung erfolgt für die Ableitung des Druckes nach der Zeit. Mithilfe eines Flutungsalgorithmus erfolgt eine Suche nach den Spitzen dieser Ableitung, gefolgt von einer Prüfung, ob es sich dabei um ein lokales Maximum handelt. Dazu wird die zweite Abteilung in einer lokalen Umgebung untersucht. Letztlich erfolgt eine signalartabhängige Prüfung auf Plausibilität.

Mit dem Ergebnis dieses ersten Schrittes erfolgt dann die Bestimmung des Anfangs und des Ende des Schlags bei den anderen Signalen.

### 2. Signalartbestimmung (fakultativ)

Auf der Grundlage der zeitlichen Definition der Herzschlaglänge werden aus den jeweiligen Drucksignalen und ihrer ersten und zweiten Ableitung nach der Zeit entsprechende Ausschnitte zur Erkennung der Art des Signals untersucht, wobei als Signalart beispielsweise der linksventrikuläre, systemisch arterielle, pulmonalarterielle, rechtsventrikuläre, rechtsatriale oder pulmonalkapilläre Verschlussdruck verwendet werden kann. Zum Zweck, der Signalarterkennung erfolgt eine Merkmalsextraktion, indem neben dem Pulsatilitätsindex, die maximale Ableitung des Drucks nach der Zeit, statistische Parameter (Mediane, Mittelwerte und Momente sowie Parameter aus der Fouriertransformation der Schlagkurve und ihrer Ableitungen bestimmt werden. Weitere Merkmale werden gewonnen aus einer Zerlegung der Kurve in zwei Hälften bzgl. der Zeitachse und/oder einer Untersuchung höherer Momente oder Kumulanten der Kurve in komplexer Darstellung. Dabei bildet die Zeitachse den Realteil und der Druck den Imaginärteil der komplexen Darstellung.

Die Bestimmung der Signalart erfolgt durch Kombination eines Scoresystems, einer stufenweisen logistischen Regression und/oder einer Diskriminanzanalyse und eines Plausibilitätsschecks. Die logistische Regression bzw. Diskriminanzanalyse benutzt sowohl Größen aus dem Zeitbereich als auch Charakteristika der Fouriertransformation des jeweiligen Schlages.

### 3. Herzzeitvolumenbestimmung

Auch die Bestimmung des Herzzeitvolumens findet auf der Basis der zeitlichen Definition einer Herzschlaglänge statt.

Dabei weist der Bestimmungsalgorithmus für eine Herzzeitvolumenbestimmung eine fakultativ messpositionsabhängige multiple Regression für Widerstand, Schlagvolumen und Herzzeitvolumen, einen Plausibilitätsscheck, sowie eine Feinkorrektur auf. Die multiple Regression benutzt sowohl Größen aus dem Zeitbereich als auch Charakteristika der Fouriertransformation des jeweiligen Schlages. Dabei handelt es sich nicht um eine Pulskonturanalyse.

### 4. Signalarttypische Auswertung

In Abhängigkeit von der Art der gemessenen Drucksignale werden spezifische hämodynamische Parameter berechnet.

### So ist für Ventrikeldrucksignale die Berechnung folgender Parameter möglich:

Systolischer, minimaler und entdiastolischer Drücke, Maximum und Minimum der Ableitung des Druckes nach der Zeit, Time Tension Index (TTI), maximale Verkürzungsgeschwindigkeit (VPM), entwickelter Druck, Pulsatilität, Relaxationszeit (tau).

Für Schlagaderdrücke können systolischer, diastolischer und mittlerer Druck, sowie Maximum und Minimum der Ableitung des Druckes nach der Zeit bestimmt werden. Weiterhin können Auswurfzeit, Pulsdruck und ein Pulsatilitätsindex berechnet werden.

Für Vorhofdrücke sind V-Welle, A-Welle, maximaler, minimaler und mittlerer Druck bestimmbar.

### 5. Niederfrequenzanalyse (Mayerwellen)

Für die Niederfrequenzanalyse wird eine Powerspektrumsschätzung mittels eines Eigenwertverfahrens (mit vier bis sechs Unterräumen) durchgeführt. Das so erhaltene Powerspektrum wird logarithmiert . Eine weitere Powerspektrumsschätzung mittels eines Eigenwertverfahrens (mit zwei Unterräumen) liefert ein stark geglättetes Spektrum, das im Wesentlichen die very-low-frequency-Komponente darstellt. Die logarithmierte very-low-frequency-Komponente wird vom logarithmierten Gesamtpowerspektrum Power subtrahiert, Dadurch wird die very-low-frequency-Komponente entfernt.

Sodann werden zwei Maxima und das dazwischen liegende Minimum identifiziert. Die Positionen der low-frequency-Komponente und der high-frequency-Komponente sind identisch mit denen des delogarithmierten Restspektrums nach Entfernung der very-low-frequency-Komponente.

Nachfolgend wird eine Delogarithmierung des Restspektrums und eine Bestimmung des Powerintegrals der low- und high-frequency-Komponenten durchgeführt.

Zur Bestimmung der Signalart wird vorteilhafterweise eine Analyse der ersten Ableitung nach der Zeit durchgeführt, wobei die Funktion f₂ des Referenzdrucksignals von der Zeit abhängig ist. Zur Berechnung des Herzzeitvolumens als hämodynamischem Parameter wird die erste und zweite Ableitung einer Funktion f₁. nach der Zeit ermittelt, wobei die Funktion f₁ der Patientendrucksignale von der Zeit abhängig ist.

Vorteilhafterweise können die bestimmten hämodynamischen Parameter auf einer Ausgabeeinheit, insbesondere einem elektronischen Datenmedium wie einer Speicherplatte, einem Netzwerk oder einem Patienteninformationssystem gespeichert und/oder auf einem Monitor dargestellt werden. Weiterhin können die Parameter direkt in ein Patienteninformationssystem übertragen werden.

In einer Ausgestaltung der Erfindung verfügt das Verfahren über ein auf einer logistischen Analyse basierenden automatischen Erkennung der Art des gemessenen Patientendruckes, insbesondere eines Druckes des linken Ventrikels, der Aorta, des rechten Ventrikels, der Pulmonalarterie, des pulmonalen Kapillarverschlussdrucks sowie des rechten Vorhofs.

Darüber hinaus wird die Aufgabe durch eine Vorrichtung mit den Merkmalen des Anspruchs 10 gelöst. Demnach ist erfindungsgemäß vorgesehen, dass eine Vorrichtung zur Bestimmung hämodynamischer Parameter aus Patientendrucksignalen
- einen Druckwandler, der in Abhängigkeit von Patientendrucksignalen elektrische Signale erzeugt,
- einen Analog/Digitalwandler, die die Signale umwandelt,
- einen Messwertempfänger, dem im Körper eines Patienten gemessene Gefäßdrücke zugeführt werden, in Form einer Signalanalyse- und Verarbeitungseinheit, die
   - die Patientendrucksignalen empfängt,
   - die ein Patientendrucksignal als Referenzdrucksignal aus den vom Messwertempfänger übermittelten Patientendrucksignalen auswählt und bestimmt,
   - die Patientendrucksignale und das Referenzdrucksignal in Segmente unterteilt.
   - die diese Segmente auf Artefakte mit Verfahren im Zeitbereich unter Verwendung der ersten Ableitung der Drucksignale nach der Zeit überprüft und artefaktbehaftete Segmente verwirft,
   - den Beginn und das Ende eines Herzschlags mit Hilfe des Refe renzdrucksignals bestimmt und
   - die nicht verworfenen Segmente der Patientendrucksignale zur Berechnung von hämodynamischen Parametern verwendet, und
- eine Ausgabeeinheit.
aufweist.

Das erfindungsgemäße Verfahren und die Vorrichtung zur Bestimmung hämodynamischer Parameter soll anhand der Zeichnungsfiguren dargestellt und näher erläutert werden. Es zeigen:
- Fig. 1: ein Blockschaltbild einer Vorrichtung, die zur Durchführung des Verfahrens zur Bestimmung hämodynamischer Parameter geeignet ist;
- Fig. 2: einen Ablaufplan des Verfahrens zur Bestimmung hämodynamischer Parameter;
- Fig. 3: eine graphische Darstellung der ersten Ableitung des Drucksignals des linken Ventrikels über der Zeit und
- Fig. 4: eine graphische Darstellung der ersten Ableitung des Drucksignals der Aorta über der Zeit.

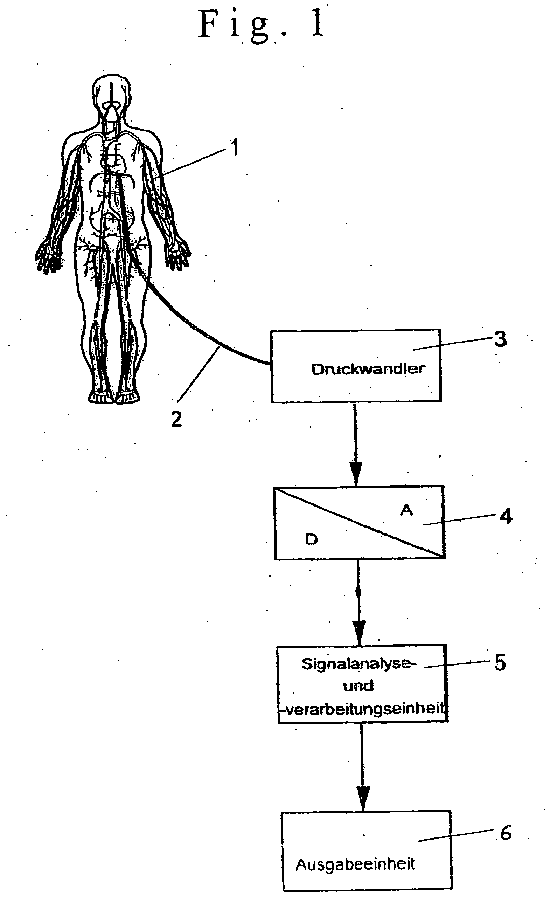

Fig. 1 zeigt einen prinzipiellen Aufbau einer invasiven Druckmessung, wobei ein das Übertragungssystem bildender Katheter 2 durch das venöse oder arterielle System eines Patienten 1 in die Nähe der Stelle bewegt wird, an der die Druckmessung vorgenommen werden soll. Ein Druckwandler 3 erzeugt in Abhängigkeit von den Patientendruckrucksignalen elektrische Signale, die einem Analog/Digitalwandler 4 zugeführt werden. Die im Analog-/Digitalwandler 4 umgewandelten Signale werden sodann an einen Messwertempfänger in Form einer Signalanalyse- und Verarbeitungseinheit 5 weitergeleitet, in der das erfindungsgemäße Verfahren zur Bestimmung hämodynamischer Parameter durchgeführt wird. Die berechneten hämodynamischen Parameter werden dann an eine Ausgabeeinheit 6, insbesondere in Form eines elektronischen Datenmediums oder Monitors weitergeleitet.

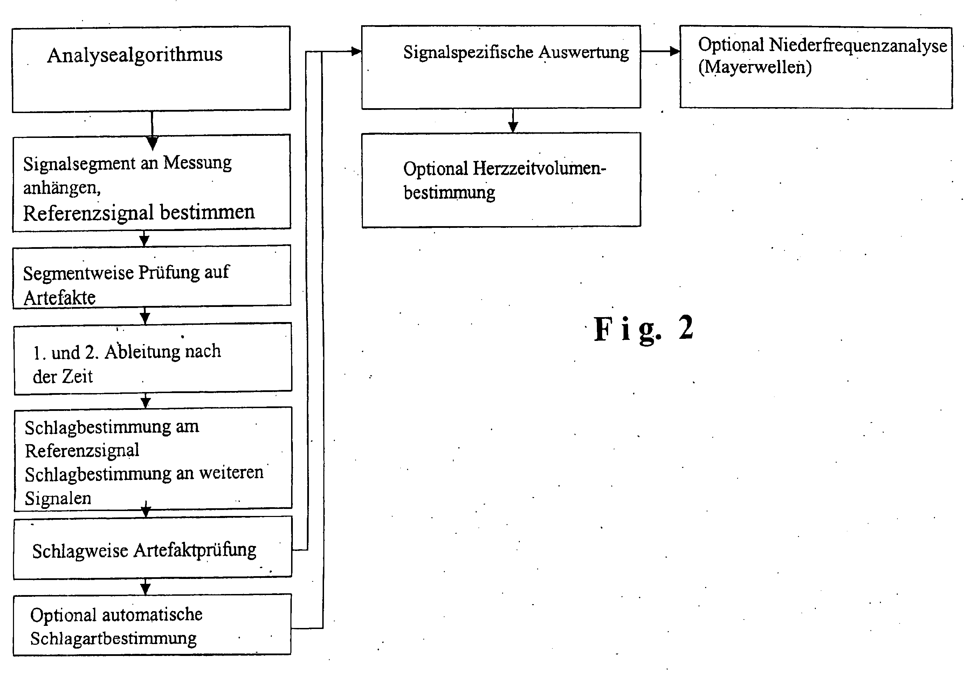

In Fig. 2 ist ein Ablaufplan des Verfahrens zur Bestimmung hämodynamischer Parameter dargestellt.

Nach diesem Verfahren wird aus den empfangenen Patientendrucksignalen zunächst eine Auswahl eines Patientendrucksignals als Refererizdrucksignal vorgenommen. Nach einer Unterteilung der Patientendrucksignale und des Referenzdrucksignals in Segmente wird eine Prüfung dieser Segmente auf Artefakte mit Verfahren im Zeit- und ggf. Frequenzbereich durchgeführt. Dazu wird die erste und zweite Ableitung der Drucksignale nach der Zeit bestimmt.

Sodann wird eine Schlagbestimmung am Referenzsignal und/oder an weiteren Signalen durchgeführt. Auf der Grundlage der Länge eines Herzschlages wird nachfolgend eine schlagweise Prüfung auf Artefakte durchgeführt. Derartige Artefakte sind z. B. Bewegungsartefakte, die durch Manipulation am Katheter, Anstoßen des Katheters im Gefäß, Unterbrechung der Druckmessung bei Katheterwechsel, Injektionen oder Spülung des Systems entstehen. Solche Segmente, die als artefaktbehaftet identifiziert sind, werden verworfen. In optionaler Weise kann nunmehr eine automatische Schlagartbestimmung durchgeführt werden.

Auf der Grundlage der schlagweisen Artefaktprüfung erfolgt eine signalspezifische Auswertung. Optional wird eine Herzzeitvolumenbestimmung und/oder eine Niederfrequenzanalyse durchgeführt.

Die berechneten hämodynamischen Parameter können auf einer Ausgabeeinheit, z. B. einem Monitor, dargestellt werden. In einer vorteilhaften Ausführungsform der Erfindung können die Ergebnisse in eine digitale Patientenakte und/oder eine automatische Befunderstellung bzw. Textverarbeitung zur Arztbrieferstellung integriert werden.

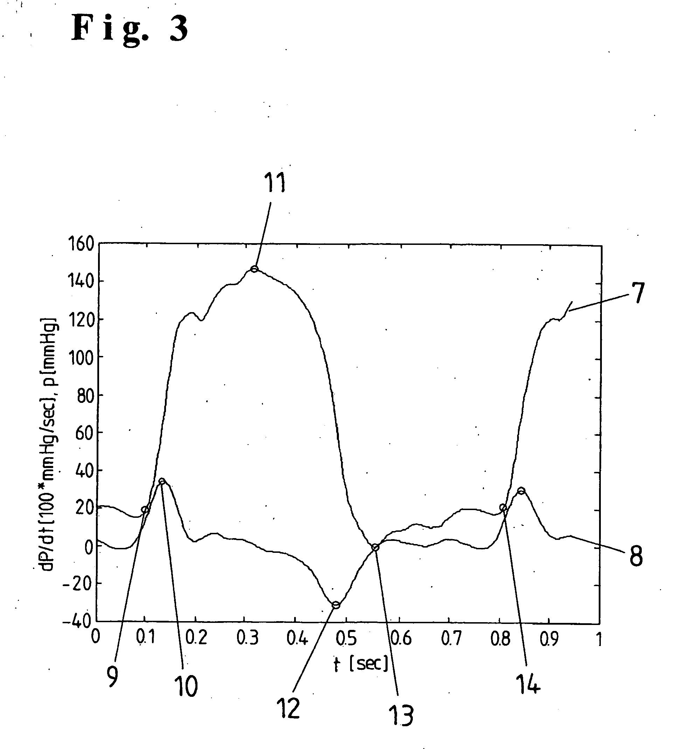

Fig. 3 zeigt eine graphische Darstellung eines linksventrikulären (LV) Patientendrucksignals 7 und der zugehörigen ersten Ableitung 8 über der Zeit. Auf der Abszisse ist die Zeit in Sekunden aufgetragen, auf der Ordinate der Druck in Millimeter Quecksilbersäule (mmHg) und die erste Ableitung des Druckes nach der Zeit in Millimeter Quecksilbersäule pro Sekunde (mmHg/sec), wobei aus Darstellungsgründen mit dem Faktor 0,01 skaliert wurde.

Mit Hilfe des erfindungsgemäßen Verfahrens werden verschiedene hämodynamische Parameter bestimmt. Dabei werden Patientendrucksignale von einem Messwertempfänger empfangen, der Gefäßdrücke im Körper eines Patienten misst.

Nach Prüfung auf (grobe) Artefakte werden nur Patientendrucksignalsegmente mit ausreichender Qualität analysiert. Nach Auswahl und Bestimmung eines Patientendrucksignals als Referenzdrucksignal aus den vom Messwertempfänger übermittelten Patientendrucksignalen 7 wird der Zeitbereich eines Herzschlags gefunden und in einer Umgebung dieser Zeit Anfang und Ende des Herzschlags am aktuellen Patientendrucksignal bestimmt. Im dargestellten Fall war das Referenzsignal ein Femoraldrucksignal.

Der Herzkammerschlag geht einem Schlag dieses Referenzsignals voraus. Es wird das Maximum der ersten Ableitung des Herzkammerdrucksignalschlags in einer zeitlichen Umgebung (z. B. 100 ms) des Maximums der ersten Ableitung des Femoraldrucksignalschlags gesucht und dann ausgehend von diesem Punkt der Beginn des steilen Anstiegs des Herzkammerdrircksignals gesucht (Enddiastole des vorangegangenen Schlags, 9). Das Ende 10 des Schlags wird genau so bestimmt. Nach Bestimmung des Herzschlags am aktuellen Drucksignal erfolgt noch einmal eine schlagweise Prüfung auf Artefakte.

Die nicht verworfenen Patientendrucksignale werden zur Berechnung von hämodynamischen Parametern verwendet. Die Kurve zeigt das Ergebnis einer automatischen Analyse. Der Schlag beginnt am Anstieg der Druckkurve mit der Enddiastole des vorangegangen Schlags 9. Das Maximum der Ableitung 10 findet sich an der Stelle des steilsten Anstiegs. Als nächste Punkte in zeitlicher Reihenfolge folgen das systolische Maximum 11 des Drucks, das Minimum 12 der Ableitung am steilsten Abfall des Drucks, das Minimum des Drucks 13 im Schlagbereich und die Enddiastole 14 des aktuellen Schlags. Aus der Schlaglänge errechnet sich die Herzfrequenz (HF) zu beispielsweise 85 Schlägen pro Minute, aus dem Schlag wird weiterhin der mittlere Druck zu 67 mmHg bestimmt, der entwickelte Druck (DP) als Druckmaximum minus enddiastolischer Druck zu 127 mmHg, der dimensionslose Pulsatilitätsindex (P1) als Druckamplitude durch Mitteldruck zu 2,1999, die Relaxationszeit (tau) nach fünf verschiedenen dem Fachmann aus der Literatur bekannten Verfahren zu tau = 16 msec, die maximale Verkürzungsgeschwindigkeit (VPM) nach einer dem Fachmann ebenfalls bekannten Berechnung zu VPM = 2,8478 Hz und der Time Tension Index. (TTI) als Integral des Drucks in der Systole zu TTI = 44,7269 mmHg*sec.

Die berechneten Parameter können dann auf einer Ausgabeeinheit 6 als Datei, am Bildschirm oder an einem Monitor dargestellt werden.

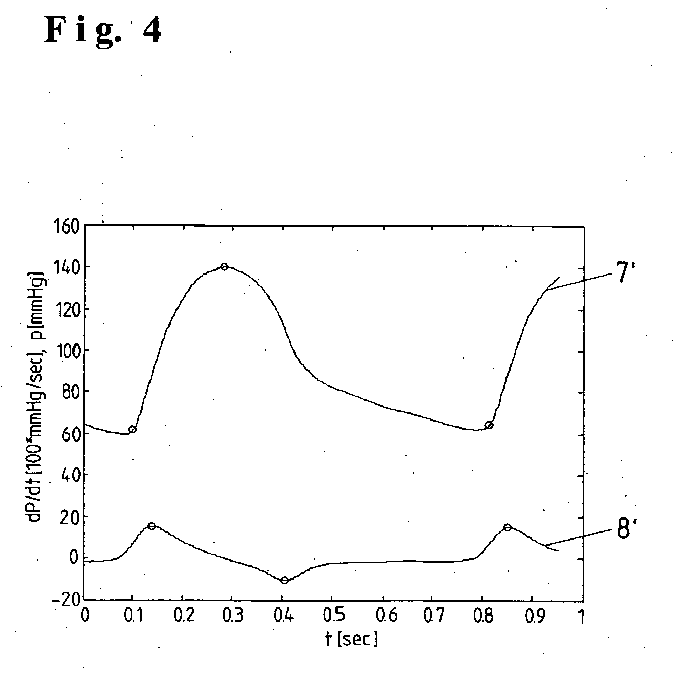

Fig. 4 zeigt eine graphische Darstellung eines Patientendrucksignals 7' aus der Aorta (AO) und der zugehörigen ersten Ableitung 8' über der Zeit. Auf der Abszisse ist die Zeit in Sekunden aufgetragen, auf der Ordinate der Druck in Millimeter Quecksilbersäule (mmHg) und die erste Ableitung des Druckes nach der Zeit in Millimeter Qecksilbersäule pro Sekunde (mmHg/sec), wobei aus Darstellungsgründen mit dem Faktor 0,01 skaliert wurde.

Die Schlagerkennung, automatische Analyse und Darstellung entspricht der in Verbindung mit Fig. 2 beschriebenen Schlagerkennung, automatische Analyse und Darstellung. Statt dem entwickelten Druck wird bei arteriellen Drücken der Pulsdruck (PP), außerdem und die Auswurfszeit (ET) in Millisekunden (msec) bestimmt. Fakultativ ist eine Berechnung des Schlagvolumens (ml/min), des Herzeitvolumenns (CO) in Liter pro Minute (l/min) und des Systemwiderstands (Res) vorgesehen.

Im gezeigten Beispiel werden die folgenden hämodynamischen Parameter berechnet: Der Pulsdruck wird zu PP = 76 mmHg bestimmt, die Auswurfszeit beträgt ET = 306 msec, das Herzeitvolumen CO = 7 l/min und der Systemwiderstand ergibt sich zu Res = 13,4 Wood Einheiten. Weiterhin wird eine Herzfrequenz HF von 84 Schläge pro Minuten berechnet. Der mittlere Druck wird zu 94 mmHg bestimmt und der dimensionslose Pulsatilitätsindex (PI) wird zu 0,83076 ermittelt.

## Patentansprüche

1. Verfahren zur Bestimmung hämodynamischer Parameter aus Patientendrucksignalen (7, 7'), wobei das Verfahren die Schritte umfasst:
a) Empfangen von Patientendrucksignalen (7, 7') von einem Messwertempfänger (5), dem im Körper (1) eines Patienten gemessene Gefäßdrücke zugeführt werden,
b) Auswahl und Bestimmung eines Patientendrucksignals als Referenzdrucksignal aus den vom Messwertempfänger (5) übermittelten Patientendrucksignalen (7, 7'),
c) Unterteilung der Patientendrucksignale (7, 7') und des Referenzdrucksignals in Segmente,
d) Prüfung dieser Segmente auf Artefakte mit Verfahren im Zeitbereich unter Verwendung der ersten Ableitung (8, 8') der Drucksignale nach der Zeit, wobei artefaktbehaftete Segmente verworfen werden,
e) Bestimmung von Beginn und Ende eines Herzschlags mit Hilfe des Referenzdrucksignals und
f) Verwendung der nicht verworfenen Segmente der Patientendrucksignale (7, 7') zur Berechnung von hämodynamischen Parametern.

2. Verfahren nach Anspruch 1, **dadurch gekennzeichnet, dass** die Prüfung der Segmente auf Artefakte mit Verfahren im Zeit- und Frequenzbereich durchgeführt wird.

3. Verfahren nach Anspruch 1 oder 2, **dadurch gekennzeichnet, dass** zur Bestimmung der Signalart eine Analyse der Ableitungen nach der Zeit durchgeführt wird.

4. Verfahren nach Anspruch 1 oder 2, **dadurch gekennzeichnet, dass** zur Bestimmung der Signalart eine Diskriminanzanalyse oder logistische Regression basierend auf schlagweiser Analyse der Druckkurve im Zeit- und Frequenzbereich durchgeführt wird.

5. Verfahren nach mindestens einem der vorangehenden Ansprüche, **dadurch gekennzeichnet, dass** zur Berechnung des Schlagvolumens als hämodynamischem Parameter eine schlagweise multiple Regression basierend auf Parametern aus normalisierten Fourierspektren, und Analysen der Druckkurve und ihrer Ableitungen im Zeitbereich vorgenommen wird.

6. Verfahren nach Anspruch 5, **dadurch gekennzeichnet, dass** zur Kalibration des Schlagvolumens als hämodynamischem Parameter Größe oder Gewicht oder Geschlecht oder Alter oder eine Kombination hiervon verwendet wird.

7. Verfahren nach mindestens einem der vorangehenden Ansprüche, **dadurch gekennzeichnet, dass** eine signalarttypische Auswertung durchgeführt wird.

8. Verfahren nach mindestens einem der vorangehenden Ansprüche, **dadurch gekennzeichnet, dass** eine Niederfrequenzanalyse durchgeführt wird.

9. Verfahren nach mindestens einem der vorangehenden Ansprüche, **dadurch gekennzeichnet, dass** die berechneten hämodynamischen Parameter elektronisch gespeichert oder auf einer Ausgabeeinheit (6), insbesondere einem Monitor, dargestellt werden.

10. Vorrichtung zur Bestimmung hämodynamischer Parameter aus Patientendrucksignalen (7, 7') mit
- einem Druckwandler (3), der in Abhängigkeit von Patientendrucksignalen elektrische Signale erzeugt,
- einem Analog/Digitalwandler (4), die die Signale umwandelt,
- einem Messwertempfänger, dem im Körper (1) eines Patienten gemessene Gefäßdrücke zugeführt werden, in Form einer Signalanalyse- und Verarbeitungseinheit (5), die
- die Patientendrucksignalen (7, 7') empfängt,
- die ein Patientendrucksignal als Referenzdrucksignal aus den vom Messwertempfänger (5) übermittelten Patientendrucksignalen (7, 7') auswählt und bestimmt,
- die Patientendrucksignale (7, 7') und das Referenzdrucksignal in Segmente unterteilt,
- die diese Segmente auf Artefakte mit Verfahren im Zeitbereich unter Verwendung der ersten Ableitung (8, 8') der Drucksignale nach der Zeit überprüft und artefaktbehaftete Segmente verwirft,
- den Beginn und das Ende eines Herzschlags mit Hilfe des Referenzdrucksignals bestimmt und
- die nicht verworfenen Segmente der Patientendrucksignale (7, 7') zur Berechnung von hämodynamischen Parametern verwendet, und
- einer Ausgabeeinheit (6).

11. Vorrichtung nach Anspruch 10, **dadurch gekennzeichnet, dass** die Signal-analyse- und Verarbeitungseinheit (5) die Segmente auf Artefakte mit Verfahren im Zeit- und Frequenzbereich prüft.

12. Vorrichtung nach Anspruch 10 oder 11, **dadurch gekennzeichnet, dass** die Signal-analyse- und Verarbeitungseinheit (5) zur Bestimmung der Signalart eine Analyse der Ableitungen nach der Zeit durchführt.

13. Vorrichtung nach Anspruch 10 oder 11, **dadurch gekennzeichnet, dass** die Signal-analyse- und Verarbeitungseinheit (5) zur Bestimmung der Signalart eine Diskriminanzanalyse oder logistische Regression basierend auf schlagweiser Analyse der Druckkurve im Zeit- und Frequenzbereich durchführt.

14. Vorrichtung nach mindestens einem der vorangehenden Ansprüche, **dadurch gekennzeichnet, dass** die Signal-analyse- und Verarbeitungseinheit (5) zur Berechnung des Schlagvolumens als hämodynamischem Parameter eine schlagweise multiple Regression basierend auf Parametern aus normalisierten Fourierspektren und Analysen der Druckkurve und ihrer Ableitungen im Zeitbereich vornimmt.

15. Vorrichtung nach Anspruch 14, **dadurch gekennzeichnet, dass** die Signal-analyse- und Verarbeitungseinheit (5) zur Kalibration des Schlagvolumens als hämodynamischem Parameter Größe oder Gewicht oder Geschlecht oder Alter oder eine Kombination hiervon verwendet.

16. Vorrichtung nach mindestens einem der vorangehenden Ansprüche, **dadurch gekennzeichnet, dass** die Signal-analyse- und Verarbeitungseinheit (5) eine signalarttypische Auswertung durchführt.

17. Vorrichtung nach mindestens einem der vorangehenden Ansprüche, **dadurch gekennzeichnet, dass** die Signal-analyse- und Verarbeitungseinheit (5) eine Niederfrequenzanalyse durchführt.

18. Vorrichtung nach mindestens einem der vorangehenden Ansprüche, **dadurch gekennzeichnet, dass** die Ausgabeeinheit (6) die berechneten hämodynamischen Parameter elektronisch speichert oder insbesondere auf einem Monitor darstellt.

## Claims

1. Method for determining haemodynamic parameters from patient pressure signals (7, 7') wherein the method comprises the steps:
a) receiving patient pressure signals (7, 7') from a measurement receiver (5) to which vessel pressures measured in the body (1) of a patient are supplied,
b) selecting and determining a patient pressure signal as reference pressure signal from the patient pressure signals (7, 7') sent from the measurement receiver (5)
c) dividing the patient pressure signals (7, 7') and reference pressure signal into segments,
d) examining these segments for artefacts with methods in the time range by using the first derivative (8,8') with respect to time of the pressure signals wherein artefact-afflicted segments are discarded,
e) determining the start and end of a heart beat by means of the reference pressure signal and
f) using the non-discarded segments of the patient pressure signals (7, 7') for calculating haemodynamic parameters.

2. Method according to claim 1 **characterised in that** the examination of the segments for artefacts is carried out with methods in the time and frequency range.

3. Method according to claim 1 or 2, **characterised in that** an analysis of the derivatives with respect to time is carried out for determining the signal type.

4. Method according to claim 1 or 2, **characterised in that** for determining the signal type a discriminant analysis or logistic regression is carried out based on beat-wise analysis of the pressure curve in the time and frequency range.

5. Method according to at least one of the preceding claims, **characterised in that** to calculate the beat volume as haemodynamic parameter a beat-wise multiple regression based on parameters from standardised Fourier spectra and analysis of the pressure curve and their derivatives is undertaken in the time range.

6. Method according to claim 5, **characterised in that** the size or weight or sex or age or a combination thereof is used for calibrating the beat volume as haemodynamic parameter.

7. Method according to at least one of the preceding claims **characterised in that** an evaluation typical of the signal-type is carried out.

8. Method according to at least one of the preceding claims, **characterised in that** a low frequency analysis is carried out.

9. Method according to at least one of the preceding claims **characterised in that** the calculated haemodynamic parameters are stored electronically or displayed on an output unit (6), more particularly a monitor.

10. Apparatus for determining haemodynamic parameters from patient pressure signals (7, 7') with
- a pressure converter (3) which produces electrical signals in accordance with patient pressure signals,
- an analog/digital converter which convert the signals,
- a measurement receiver, which is supplied with vessel pressures measured in the body (1) of a patient, in the form of a signal analysis and treatment unit (5), which
- receives the patient pressure signals (7, 7'),
- selects and determines a patient pressure signal as reference pressure signal from the patient pressure signals (7, 7') sent from the measurement receiver (5)
- divides the patient pressure signals (7, 7') and reference pressure signal into segments,
- examines these segments for artefacts with methods in the time range by using the first derivative (8,8') with respect to time of the pressure signals wherein artefact-afflicted segments are discarded,
- determines the start and end of a heart beat by means of the reference pressure signal and
- uses the non-discarded segments of the patient pressure signals (7, 7') for calculating haemodynamic parameters, and
an output unit (6).

11. Apparatus according to claim 10 **characterised in that** the signal analysis and treatment unit (5) carries out the examination of the segments for artefacts with methods in the time and frequency range.

12. Apparatus according to claim 10 or 11, **characterised in that** the signal analysis and treatment unit (5) carries out an analysis of the derivatives with respect to time for determining the signal type.

13. Apparatus according to claim 10 or 11, **characterised in that** the signal analysis and treatment unit (5) carries out a discriminant analysis or logistic regression for determining the signal type based on beat-wise analysis of the pressure curve in the time and frequency range.

14. Apparatus according to at least one of the preceding claims, **characterised in that** the signal analysis and treatment unit (5) undertakes a beat-wise multiple regression based on parameters from standardised Fourier spectra and analyses of the pressure curve and their derivatives in the time range to calculate the beat volume as haemodynamic parameter.

15. Apparatus according to claim 14, **characterised in that** the signal analysis and treatment unit (5) uses the size or weight or sex or age or a combination thereof for calibrating the beat volume as haemodynamic parameter.

16. Apparatus according to at least one of the preceding claims **characterised in that** the signal analysis and treatment unit (5) carries out an evaluation typical of the signal-type.

17. Apparatus according to at least one of the preceding claims, **characterised in that** the signal analysis and treatment unit (5) carries out a low frequency analysis.

18. Apparatus according to at least one of the preceding claims **characterised in that** the output unit (6) stores electronically or displays particularly on a monitor the calculated haemodynamic parameters.

## Revendications

1. Procédé pour déterminer des paramètres hémodynamiques à partir de signaux de pression d'un patient (7, 7'), le procédé comprenant les étapes suivantes :
a) la réception de signaux de pression du patient (7, 7') depuis un récepteur de valeurs mesurées (5) auquel des pressions vasculaires mesurées dans le corps (1) d'un patient sont transmises,
b) la sélection et la détermination d'un signal de pression du patient comme signal de pression de référence à partir des signaux de pression du patient (7, 7') transmis par le récepteur de valeurs mesurées (5),
c) la division des signaux de pression du patient (7, 7') et du signal de pression de référence en segments,
d) le contrôle de la présence d'artefacts dans ces segments avec des procédés dans la plage de temps en utilisant la première dérivée (8, 8') des signaux de pression par rapport au temps, les segments présentant des artefacts étant rejetés,
e) la détermination du début et de la fin d'un battement cardiaque à l'aide du signal de pression de référence et
f) l'utilisation des segments non rejetés des signaux de pression du patient (7, 7') pour calculer des paramètres hémodynamiques.

2. Procédé selon la revendication 1, **caractérisé en ce que** le contrôle de la présence d'artefacts dans les segments est effectué avec des procédés dans les plages de temps et de fréquence.

3. Procédé selon la revendication 1 ou 2, **caractérisé en ce que**, pour déterminer le type de signal, une analyse des dérivées par rapport au temps est effectuée.

4. Procédé selon la revendication 1 ou 2, **caractérisé en ce que**, pour déterminer le type de signal, une analyse discriminatoire ou une régression logistique est effectuée sur la base d'une analyse à chaque battement de la courbe de pression dans les plages de temps et de fréquence.

5. Procédé selon au moins l'une quelconque des revendications précédentes, **caractérisé en ce que**, pour calculer le volume de battement en tant que paramètre hémodynamique, une régression multiple est effectuée à chaque battement sur la base de paramètres issus de spectres de Fourier normalisés et d'analyses de la courbe de pression et de ses dérivées dans la plage de temps.

6. Procédé selon la revendication 5, **caractérisé en ce que**, pour calibrer le volume du battement en tant que paramètre hémodynamique, on utilise la taille, le poids, le sexe ou l'âge ou bien une combinaison de ceux-ci.

7. Procédé selon au moins l'une quelconque des revendications précédentes, **caractérisé en ce qu'**une évaluation typique pour le type de signal est effectuée.

8. Procédé selon au moins l'une quelconque des revendications précédentes, **caractérisé en ce qu'**une analyse basse fréquence est effectuée.

9. Procédé selon au moins l'une quelconque des revendications précédentes, **caractérisé en ce que** les paramètres hémodynamiques calculés sont mémorisés électroniquement ou représentés sur une unité de sortie (6), plus particulièrement sur un moniteur.

10. Dispositif pour déterminer des paramètres hémodynamiques à partir de signaux de pression du patient (7, 7') avec
- un convertisseur de pression (3) qui génère des signaux électriques en fonction des signaux de pression du patient,
- un convertisseur analogique/numérique (4) qui transforme les signaux,
- un récepteur de valeurs mesurées auquel des pressions vasculaires mesurées dans le corps (1) d'un patient sont transmises, sous la forme d'une unité d'analyse et de traitement de signaux (5) qui
- reçoit les signaux de pression du patient (7, 7'),
- sélectionne et détermine un signal de pression du patient comme signal de pression de référence à partir des signaux de pression du patient (7, 7') transmis par le récepteur de valeurs mesurées (5),
- divise les signaux de pression du patient (7, 7') et le signal de pression de référence en segments,
- vérifie la présence d'artefacts dans ces segments avec des procédés dans la plage de temps en utilisant la première dérivée (8, 8') des signaux de pression par rapport au temps et rejette les segments présentant des artefacts,
- détermine le début et la fin d'un battement cardiaque à l'aide du signal de pression de référence et
- utilise les segments non rejetés des signaux de pression du patient (7, 7') pour calculer des paramètres hémodynamiques et
- une unité de sortie (6).

11. Dispositif selon la revendication 10, **caractérisé en ce que** l'unité d'analyse et de traitement de signaux (5) contrôle la présence d'artefacts dans les segments avec des procédés dans les plages de temps et de fréquence.

12. Dispositif selon la revendication 10 ou 11, **caractérisé en ce que** l'unité d'analyse et de traitement de signaux (5) effectue une analyse des dérivées par rapport au temps pour déterminer le type de signal.

13. Dispositif selon la revendication 10 ou 11, **caractérisé en ce que**, pour déterminer le type de signal, l'unité d'analyse et de traitement de signaux (5) effectue une analyse discriminatoire ou une régression logistique sur la base d'une analyse à chaque battement de la courbe de pression dans les plages de temps et de fréquence.

14. Dispositif selon au moins l'une quelconque des revendications précédentes, **caractérisé en ce que**, pour calculer le volume de battement comme paramètre hémodynamique, l'unité d'analyse et de traitement de signaux (5) effectue à chaque battement une régression multiple basée sur des paramètres issus de spectres de Fourier normalisés et d'analyses de la courbe de pression et de ses dérivées dans la plage de temps.

15. Dispositif selon la revendication 14, **caractérisé en ce**, pour calibrer le volume de battement comme paramètre hémodynamique, l'unité d'analyse et de traitement de signaux (5) utilise la taille, le poids, le sexe ou l'âge ou bien une combinaison de ceux-ci.

16. Dispositif selon au moins l'une quelconque des revendications précédentes, **caractérisé en ce que** l'unité d'analyse et de traitement de signaux (5) effectue une évaluation typique pour le type de signal.

17. Dispositif selon au moins l'une quelconque des revendications précédentes, **caractérisé en ce que** l'unité d'analyse et de traitement de signaux (5) effectue une analyse basse fréquence.

18. Dispositif selon au moins l'une quelconque des revendications précédentes, **caractérisé en ce que** l'unité de sortie (6) mémorise électroniquement les paramètres hémodynamiques calculés ou, plus particulièrement, les représente sur un moniteur.
